# EUROPEAN PATENT SPECIFICATION

(11) **EP 3 934 621 B1**
(45) Date of publication and mention of the grant of the patent: **25.06.2025**
(21) Application number: 20718768.3
(22) Date of filing: 09.03.2020
(51) Int. Cl.: A61K 9/00, A61K 9/06, A61K 31/05, A61K 31/164, A61K 47/06, A61K 47/44

(54) **PREPARATION FOR TOPICAL USE FOR DERMATOLOGICAL TREATMENTS**
ZUBEREITUNG ZUR TOPISCHEN ANWENDUNG FÜR DERMATOLOGISCHE BEHANDLUNGEN
PRÉPARATION À USAGE TOPIQUE POUR TRAITEMENTS DERMATOLOGIQUES

(30) Priority: 07.03.2019 IT 201900003349; 09.08.2019 IT 201900014568
(43) Date of publication of application: 12.01.2022
(73) Proprietor: Hug Research Labs Srl, 17100 Savona (IT)
(72) Inventor: BOZZOLASCO, Selena, 17011 Ceva (IT)
(74) Representative: Bottino, Giovanni
(86) International application number: PCT/IB2020/052022
(87) International publication number: WO 2020/178805

(56) References cited:
- WO-A1-2016/103254
- ANONYMOUS: "Understanding what you'll find in CBD: Terpenes", 1 March 2019 (2019-03-01), pages 1 - 10, XP055634878, Retrieved from the Internet <URL:https://www.frontierjackson.com/blog/cbd/what-are-terpenes/> [retrieved on 20191022]
- ANONYMOUS: "Know the Power of Ozonated CBD Skin Creams || Ozonated Oils", 8 August 2018 (2018-08-08), pages 1 - 8, XP055634863, Retrieved from the Internet <URL:https://organichempoil.shop/ozonated-oils/> [retrieved on 20191022]
- ETHAN B RUSSO: "Taming THC: potential cannabis synergy and phytocannabinoid-terpenoid entourage effects : Phytocannabinoid-terpenoid entourage effects", BRITISH JOURNAL OF PHARMACOLOGY, vol. 163, no. 7, 12 July 2011 (2011-07-12), UK, pages 1344 - 1364, XP055420723, ISSN: 0007-1188, DOI: 10.1111/j.1476-5381.2011.01238.x
- LUIGI ROMANO ET AL: "An Overview of Galenic Preparation Methods for Medicinal Cannabis", CURRENT BIOACTIVE COMPOUNDS, vol. 14, June 2018 (2018-06-01), pages 1 - 20, XP055634721, ISSN: 1573-4072, DOI: 10.2174/1573407214666180612080412
- PAOLA DE OLIVEIRA ET AL: "Ozonolysis of neem oil: preparation and characterization of potent antibacterial agents against multidrug resistant bacterial strains", RSC ADVANCES, vol. 7, no. 55, January 2017 (2017-01-01), pages 34356 - 34365, XP055634882, DOI: 10.1039/C7RA00574A

## Description

The present invention relates to a preparation for topical use for dermatological treatments comprising Cannabidiol as the main active ingredient.

In particular, the invention refers to a cosmetic-pharmaceutical field, a cosmeceutical field, which involves not only the treatment of diseases, but also aesthetic care: what in the last century was not within the health scope, now in fact is considered as an actual disease, or at least as a condition to be treated as it would be for a diabetes or an ulcer.

The World Health Organization maintains that the concept of health must be understood as "a state of complete physical, mental and social well-being and not merely the absence of disease", precisely to emphasize the concept that it is not the absence of disease that establishes that an organism is healthy, but rather a general well-being.

Particularly felt is the problem of wrinkles, whose appearance represents the sign of skin aging and photo-damage due to sunlight.

The skin is made up of various elements including the epidermis and the dermis: the first performs a protective function of the body while the second acts as a support to the epidermis and is responsible for the tone of the skin.

The compact structure of the dermis is due to the presence of collagen and elastic fibres, the loss of which is one of the main aging factors. Over time, due to alterations in tone and elasticity, the skin is no longer able to release itself and the first depressions remain, which progressively evolve with skin aging.

The contraction of the facial muscles is another essential and predominant factor in wrinkle formation. The chronic contraction of the muscles of the face forms, over time, visible folds on the face even when it is relaxed and over the years the wrinkles tend to remain even "at rest".

To significantly reduce wrinkles and find smoother skin, it is necessary to act on the main mechanism responsible for wrinkle formation and promote skin decontraction.

Various preparations are known in the state of the art which involve the use of cannabinoid substances, such as cannabidiol, as an active ingredient, to relax the facial muscles and limit the formation of wrinkles.

The main disadvantage of the preparations known to the state of the art is linked to the absorption of the active ingredient, as these preparations do not allow an absorption which allows an effective anti-wrinkle action.

To solve this problem, the trend in preparations known in the state of the art is to increase the dose of active ingredient, but it is evident that this solution is difficult to sustain, both from a preparation and an economic point of view.

There is therefore a need, not met by preparations known in the state of the art, to obtain a preparation which has a composition such as to guarantee a high absorption of the active ingredient used, increasing the effectiveness of the preparation itself.

The present invention achieves the above objects by realizing a preparation as described above, in which synthetic Cannabidiol is provided in association with one or more ingredients selected from the group of terpenes, characterized in that the preparation further comprises unsaturated oils capable of increasing the cutaneous absorption of cannabidiol, said oils comprising one or more ozonized oily components.

On a natural level, cannabidiol inside the plant consists of a phytocomplex, that is, cannabidiol is present in combination with terpenes, biomolecules made up of multiples of the isoprenic unit, obtained in biological systems by the reaction of several units of IPP (isopentenyl pyrophosphate) and DMAPP (dimethylallyl pyrophosphate), which have the function of enhancing the active ingredient.

The preparation object of the present invention therefore aims at recreating the environment of the phytocomplex, providing both cannabidiol and terpenes as components, in order to enhance the effect of cannabidiol.

According to this configuration, and thanks to features that will be described later, the preparation object of the present invention not only has an anti-wrinkle effect, but allows to treat dermatitis and skin inflammation and is particularly effective against the secretion of substances that generate acne.

Unlike preparations known in the state of the art, such as for example the preparation described in document WO2016/103254, the preparation object of the present invention is not related to a drug, but to a preparation for cosmetic use.

The difference is substantial, as the drugs that use cannabinoid substances use the inflorescence of the cannabis plant, which is extracted in oil and from this extraction the active ingredients come out, such as Delta-9-tetrahydrocannabinol (THC) and Cannabidiol (CBD).

The preparation of the present invention, on the other hand, does not provide for the extraction of cannabinoid substances, but aims at creating a cosmetic that reproduces the conditions of a plant.

In particular, the preparation object of the present invention uses synthetic cannabidiol for cosmetic use, which is not extracted from the plant.

In fact, the Cannabidiol extracted from the plant cannot be used in cosmetics, since when it is extracted, parts of Delta-9-tetrahydrocannabinol (THC), an active ingredient that cannot be used in cosmetics as provided for by law, are dragged by it.

As will be seen later, the use of synthetic Cannabidiol requires the reproduction of the phytocomplex, that is the environment in which Cannabidiol is naturally found, to maximize its beneficial effects.

Advantageously, the terpenes belonging to the preparation object of the present invention, are not extracted from the cannabis plant, but are extracted from plants that contain terpenes present in cannabis, such as for example pine, black pepper, Citrus Aurantium Dulcis, lavender, Cymbopogon.

In addition to enhancing the effect of the active principle used, one of the main purposes of the present patent application is to increase the absorption of the active ingredient at the skin level.

For this reason, the preparation comprises unsaturated oils.

Cannabidiol is in fact a lipophilic substance, it is solubilized inside oily substances.

As will be described later with regard to the preparation method, cannabidiol is not only solubilized in oily substances, but the preparation object of the present invention provides for the addition of oily substances with previously ozonized oily components.

Clinical studies have shown that the ozonation of oily substances improves the diffusion of the active ingredient, as ozone causes irrigation and dilation of vessels, improving their penetration.

In addition, ozonation stabilizes cannabidiol inside the preparation because ozone forms bonds that allow cannabidiol to be anchored to the terpenes.

Ozone is in fact a highly reactive molecule, therefore it forms stable bonds with both cannabidiol and terpenes.

As will be described later, synthetic Cannabidiol, before being mixed with the terpenes, is solubilized in unsaturated oil, which is previously ozonized, so as to increase the bond both with the Cannabidiol and with the terpenes that will be mixed.

Ozone allows to increase the capillary permeability and allows a better diffusion of the active ingredients.

Therefore, the preparation object of the present invention provides a combination of synthetic cannabidiol, terpenes and unsaturated oils comprising ozonized oily substances.

This complex allows to obtain surprising results both in terms of anti-wrinkle action and for the treatment of dermatitis, inflammation or skin diseases.

Advantageously, only a few terpenes are selected, based on their specific functions.

Preferably the following terpenes are used:
alpha-pinene, for its antimicrobial properties,
limonene, for its anti-inflammatory and regenerating properties on damaged skin tissues,
myrcene, for its anti-inflammatory action,
B-cariophyllene, for its anti-inflammatory action,
linalool, for its anti-inflammatory action and its mitigation of the effects of scars.

From the studies conducted on the preparation object of the present invention, it has been shown that the use of ozonized oily substances favours the absorption by the epidermis not only of cannabidiol, but of any active ingredient.

Preferably, a specific ozonation process can be provided, which is particularly effective using vegetable oils, such as Neem oil, peach oil, linseed oil, rosehip oil.

This process involves exposure of the oil to a continuous flow of ozone under a pulsed magnetic field at low temperatures.

Inside the applicator there must be a solenoid (a circular shape magnet) with a base diameter of a few centimetres from which a low frequency magnetic field is generated, between 1 hz and 50 hz, and at low temperature. Then an ozone flow is inserted into the oil inside the solenoid crossed by an initial direct current or a type of electric current with fixed and invariable polarity characterized by the orderly displacement of the ozone electric charges that cross the surface in the time unit. This trend is introduced with the alternating one which corresponds to an inversion of the trend of electric charges consequent to the inversion of the poles, respectively positive and negative, which occurs with a fixed frequency, around 50/60HZ. Scientific studies have shown that ozone, however highly unstable as a gas, can be trapped inside vegetable oils composed of saturated and unsaturated fatty acids that retain ozone and prolong its action. Obviously, the greater the number of unsaturated fatty acids, the greater the ability to retain ozone, which will give rise to ozonides.

Ozonide formation process:
- electrophilic attack of the unsaturated double bond to ozone

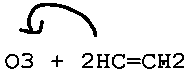

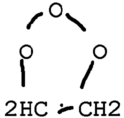

Subsequent rearrangement and formation of the stable secondary ozonide.

Consequently, the short chain of fatty acids obtained by ozone saturation, and the hydrophilicity of the oil obtained by the fragmentation given by the insertion of polar ozone, allow the new molecule to merge with the cell wall and pour into the cytosol by stimulating through a series of biochemical mechanisms the cell to protect against natural harmful substances (anti-radical action) and increasing the energy content as ATP. Therefore, the obtained ozonized oil generally has a bactericidal, antiviral, anti-inflammatory, photoprotective and repairing / regenerative action on the tissues.

This ozonized oil is then added to the mixture consisting of cannabidiol and terpenes, as will be described later.

As anticipated, on the basis of the performed experiments, unsaturated oils are to be provided in the preparation object of the present invention.

Among unsaturated oils, olive oil is the oil of choice, as well as sunflower oil.

However, since the preparation object of the present invention foresees a mainly cosmetic use, according to a possible embodiment, ozonized Neem oil is used.

Neem oil is in fact a highly unsaturated oil, which chemically has a structure similar to olive oil.

Ozonized Neem oil is not used in cosmetics because of the bad smell.

In the preparation object of the present invention, this disadvantage is solved by providing for the use of perfumed substances.

Terpenes can also be responsible for the fragrance (for example limonene gives the classic lemon perfume), but specific fragrant substances known to the state of the art can be used.

According to an improvement, Cannabidiol is present in a percentage comprised between 0.05% and 0.5% with respect to the total weight of the preparation, preferably in a percentage comprised between 0.1% and 0.3%, 0.2% in particular.

Advantageously, the ozonized oily component is present in a percentage between 0.01% and 20% of the total weight of the preparation.

The percentage of the oily component also varies according to the nature of the preparation, i.e. whether the latter is provided in the form of a gel, cream, ointment, emulsion or oil.

By way of example, preferably the ozonized oily component is present in a percentage of about 5% if the preparation is provided in the form of an oil, while it is provided in a percentage of about 0.01% if the preparation is provided in the form of a cream.

According to the ozonation process, in fact, the ozonized oil can generate unpleasant odours, a detail that should not be underestimated in cosmetics.

For this reason, the preparation object of the present invention can preferably be provided in the form of dry oil, with higher percentages of ozonized oily component, and in cream form, with lower percentages of ozonized oily component.

Since the ozonized oily component has a high anti-wrinkle power, it is possible, for example, to provide a kit including a dry oil which is applied to areas of particular muscle tension (such as the eye area and the mouth area), and a cream which is spread in the remaining parts and/or above the areas of greatest muscle tension.

Some embodiment examples of methodologies for making the preparation object of the present invention will be described later, but it is anticipated that to obtain the oil it is preferable to insert preservatives and emulsifiers, such as esters, to degrease the oil phase and make the preparation drier, while emulsifying and texturizing substances are provided to vary the texture of the cream.

Finally, according to a possible embodiment variant, the preparation object of the present invention can provide that at least the active ingredient of cannabidiol is foreseen incorporated inside niosomes.

This configuration is particularly effective for the use of the preparation object of the present invention for the treatment of pathologies, such as dermatitis or epidermal inflammation.

Since the mere presence of the ozonized oily component considerably increases the effectiveness of the active ingredient, the present disclosure also relates to a preparation for topical use for dermatological treatments comprising cannabidiol as the main active ingredient in association with one or more ozonized oily components (not claimed).

This preparation can have one or more of the features described above.

It is evident from what has just been described that the invention described in the patent application has the aim of reproducing, in a preparation for topical use, the features of the phytocomplex.

For this reason, the preparation subject of the present patent application provides the combination of synthetic cannabidiol, one or more terpenes and unsaturated oils comprising ozonized oily components.

Finally, having regard to the advantageous aspects just described, the present invention also relates to a method for preparing a preparation for topical use for dermatological treatments.

In particular, the method includes the following steps:
a) solubilization of synthetic cannabidiol inside an oily component,
b) addition of one or more ingredients chosen from the terpenes group,
c) addition of unsaturated oils comprising one or more ozonized oily components.

The just described method steps allow to realize the preparation object of the present invention, which provides a combination of synthetic cannabidiol, terpenes and unsaturated oils comprising ozonized oily components, both in the form of a cream and of an oil.

In addition to these components, as previously described, the preparation object of the present invention can present different types of excipients, which modify the preparation and processing temperatures of the preparation itself.

The effectiveness of the preparation and the success of the method object of the present invention depend strongly on the temperatures at which these steps are performed, above all for the nature of the components used.

In fact, according to a first embodiment, step b) relating to the addition of one or more ingredients selected from the group of terpenes and step c) relating to the addition of unsaturated oils comprising one or more ozonized oily components, take place at a temperature below 45 °C.

At higher temperatures, for example at temperatures necessary to make an emulsion for a cream, equal to about 70 °C, both ozone and terpenes, which are highly volatile components, would evaporate, rendering their contribution and the advantages of absorption and effectiveness of the preparation.

According to a possible implementation variant, aimed at avoiding deterioration of the active ingredient, cannabidiol, step a) relating to the solubilization of the latter, takes place at a temperature between 30 °C and 50 °C.

As anticipated, the preparation object of the present invention is preferably provided in the form of a cream or dry oil, of which two embodiment examples will be described.

In the first case, it is necessary to provide a step for realizing an emulsion and subsequent incorporation of the solubilization obtained in step a) inside the emulsion.

Advantageously, the realization of an emulsion provides the following substeps:
- heating to a temperature between 65 °C and 85 °C of a certain quantity of a lipophilic component,
- addition of the same quantity of water,
- upon reaching the phase transition temperature, adding water in order to obtain the emulsion.

Some possible embodiments and corresponding realization methods of the preparation object of the present invention will be described below.

In particular, these embodiments concern the realization of the preparation in the form of a dry oil and in the form of a cream.

These forms are not intended to be limited to the inventive concept of the present patent application, which provides for the preparation of a preparation for topical use, aimed at maximizing the action of the active ingredient, namely cannabidiol.

On the basis of what has been previously described, in fact, the active ingredient cannabidiol is provided in association with terpenes and ozonized oily substances, in combination.

### Realization of the preparation as a dry oil and composition

Weigh all the components and solubilize cannabidiol and more solid compounds under light heating and under stirring until complete dissolution. Solubilize Phosphatidylcholine in Isopropyl Myristate and ethylhexyl stearate, cool and dissolve under slow stirring. Check that the temperature is below 38 °C and add, while stirring: Gossypium herbaceum seed oil, Helianthus annum oil, Calendula officinalis extract, Chamomilla Recutita extract, Bisabolol, Vitis vinifera seed oil, Ribes nigrum seed oil, Hydrogenated ethyhexyl olivate, Hydrogenated olive oil unsaponifiable, Persea gratissima oil, Isopropyl palmitate, Phenylea Europa alcohol, Oil Unsaponifiables, Borago Officinalis Seed Oil, Dicaprylyl Ether, Fish glycerides and the perfume. Finally, introduce all the terpenes: Alpha-pinene, Limonene, Myrcene, B-Cariophyllene and Linalol, lastly and under slow stirring add the ozonized Neem oil. Mix until homogeneous. Pack.

Still using the same procedure, but by varying the ingredients, it is possible to provide the following composition:
Dicaprylyl ether, Isopropyl myristate, Ethylhexyl stearate, Hydrogenated ethylhexyl olivate, Isopropyl palmitate, Limonene, Borago officinalis seed oil, Ozonized Azadirachta indica seed oil, Ribes nigrum fruit oil, Vitis vinifera seed oil, Gossypium herbaceum seed oil, Helian europaea oil unsaponifiables, Persea gratissima oil, Parfum, Phosphatidylcholine, Bisabolol, Hydrogenated olive oil unsaponifiables, Phenethyl alcohol, Illicium verum fruit / seed oil, Calendula officinalis flower extract, Fish glycerides, Cannabidiol, Citrus nobilis peel oil, Citrus aurantium dulcis, Chamomilla recutita flower extract, Linalool, Citrus aurantium amara peel oil, Citrus grandis peel oil, Citrus aurantium bergamia fruit oil, Citral, Geraniol, Citrus limon peel oil, Beta-caryophyllene, l-Alpha-pinene, Myrcene.

The ozonized oil could also be called Ozonized neem oil and therefore should be registered with both names.

### Creation of the preparation as a cream and composition

Heat the two phases separately until dissolved.
Phase A: Ethylhexyl stearate, Cetearyl Ethylhexanoate, Isopropyl Myristate, Dicaprylyl Ether, cannabidiol, Polysorbate 80, Phenoxyethanol, Ethylhexyl glycerin, PEG-120 Methyl Glucose Dioleate, Cyclopentasiloxane, Dimethiconol, Hydrogenated ethyhexyl olivate, Hydrogenated olive oil unsaponifiable. Cool slightly and add Linum usitatissium seed oil, Ribes nigrum seed oil.
Phase B: weigh and mix Aqua, glycerin, Glucose, Sorbitol, Sodium Glutamate, Urea, Sodium PCA, Glycine, Lactic Acid, Hydrolyzed Wheat Protein, Panthenol. Join the two phases, under turbo, for the time necessary for the emulsion to take place. Add under turbo cellulose and Hydroxyethyl Acrylate/Sodium Acryloyldimethyl Taurate Copolymer. Once homogenized, add under slow stirring the perfume and terpenes: Alpha-pinene, Limonene, Myrcene, B-Cariophyllene and Linalol, lastly and under slow stirring add the ozonized Neem oil. Pack.

Still using the same procedure, but by varying the ingredients, it is possible to provide the following composition:
Aqua, Dicaprylyl ether, Hydrogenated ethylhexyl olivate, Cetearyl ethylhexanoate, Glycerin, Polysorbate 80, Ribes nigrum fruit oil, PEG-120 methyl glucose dioleate, Cellulose, Ethylhexyl stearate, Hydroxyethyl acrylate/sodium acryloyldimethyl taurate copolymer, Parfum, Phenoxyethanol, Cyclopentasiloxane, Hydrogenated olive oil unsaponifiables, Linum usitatissimum seed oil, Cannabidiol, Isopropyl myristate, Glucose, Ethylhexylglycerin, Sorbitol, Dimethiconol, Sodium glutamate, Urea, Tocopherol, Lecithin, Ascorbyl palmitate, Citric acid, Glycine, Hydrolyzed wheat protein, Lactic acid, Sodium PCA, Ozonized Azadirachta indica seed oil, Panthenol, Linalool, Beta-caryophyllene, l-Alpha-pinene, Myrcene, Limonene.

The ozonized oil could also be called Ozonized neem oil and therefore should be registered with both names.

## Claims

1. Preparation for topical use for dermatological treatments comprising synthetic Cannabidiol as the main active ingredient,
Cannabidiol being provided in association with one or more ingredients chosen from the terpenes group,
**characterized in that**
the preparation further comprises unsaturated oils capable of increasing the cutaneous absorption of Cannabidiol,
said oils comprising one or more ozonized oily components.

2. Preparation according to claim 1, wherein Cannabidiol is present in a percentage comprised between 0.05% and 0.5% of its total weight.

3. Preparation according to one or more of the preceding claims, wherein said oils comprise ozonized Neem oil.

4. Preparation according to one or more of the preceding claims, wherein the ozonized oily component is present in a percentage comprised between 0.01% and 20% of its total weight.

5. Preparation according to one or more of the preceding claims, wherein said preparation is provided in the form of gel, cream, ointment, emulsion or oil.

6. Preparation method of a preparation for topical use for dermatological treatments **characterized in that** it includes the following steps:
a) solubilization of synthetic cannabidiol inside an oily component,
b) addition of one or more ingredients chosen from the terpenes group,
c) addition of unsaturated oils comprising one or more ozonized oily components.

7. Method according to claim 6, wherein steps b) and c) take place at a temperature lower than 45 °C.

8. Method according to claim 6 or claim 7, wherein step a) takes place at a temperature comprised between 30 °C and 50 °C.

9. Method according to one or more of the preceding claims 6 to 8, wherein after step a) the following steps are provided:
- realization of an emulsion,
- incorporation of the solubilization obtained in step a) inside the emulsion.

10. Method according to one or more of claims 6 to 9, wherein the step of making an emulsion provides the following substeps:
- heating to a temperature between 65 °C and 85 °C of a certain quantity of a lipophilic component,
- addition of the same quantity of water,
- upon reaching the phase transition temperature, adding water in order to obtain the emulsion.

## Patentansprüche

1. Zubereitung zur topischen Anwendung für dermatologische Behandlungen, die synthetisches Cannabidiol als Hauptwirkstoff umfasst,
wobei Cannabidiol in Verbindung mit einem oder mehreren Inhaltsstoffen, ausgewählt aus der Gruppe der Terpene, bereitgestellt ist,
**dadurch gekennzeichnet, dass**
die Zubereitung weiter ungesättigte Öle umfasst, die in der Lage sind, die kutane Absorption von Cannabidiol zu erhöhen,
wobei die Öle eine oder mehrere ozonisierte ölige Komponenten umfassen.

2. Zubereitung nach Anspruch 1, wobei Cannabidiol in einem Prozentsatz zwischen 0,05 % und 0,5 % seines Gesamtgewichts vorliegt.

3. Zubereitung nach einem oder mehreren der vorstehenden Ansprüche, wobei die Öle mindestens ozoniertes Neemöl umfassen.

4. Zubereitung nach einem oder mehreren der vorstehenden Ansprüche, wobei das ozonisierte Öl in einem Prozentsatz zwischen 0,01 % und 20 % bereitgestellt ist.

5. Zubereitung nach einem oder mehreren der vorstehenden Ansprüche, wobei die Zubereitung in Form von Gel, Creme, Salbe, Emulsion oder Öl bereitgestellt wird.

6. Herstellungsverfahren einer Zubereitung zur topischen Anwendung für dermatologische Behandlungen, **dadurch gekennzeichnet, dass** es die folgenden Schritte einschließt:
a) Solubilisierung von synthetischem Cannabidiol in einer öligen Komponente,
b) Zugabe eines oder mehrerer Inhaltsstoffe ausgewählt aus der Gruppe der Terpene,
c) Zugabe von ungesättigten Ölen, die eine oder mehrere ozonisierte Ölkomponenten umfassen.

7. Verfahren nach Anspruch 6, wobei die Schritte b) und c) bei einer Temperatur niedriger als 45 °C erfolgen.

8. Verfahren nach Anspruch 6 oder Anspruch 7, wobei Schritt a) bei einer Temperatur zwischen 30 °C und 50 °C stattfindet.

9. Verfahren nach einem oder mehreren der vorstehenden Ansprüche 6 bis 8, wobei nach Schritt a) die folgenden Schritte bereitgestellt werden:
- Herstellung einer Emulsion,
- Einarbeiten der in Schritt a) erhaltenen Solubilisierung in die Emulsion.

10. Verfahren nach einem oder mehreren der Ansprüche 6 bis 9, wobei der Schritt des Herstellen einer Emulsion die folgenden Teilschritte bereitstellt:
- Erhitzen einer bestimmten Menge einer lipophilen Komponente auf eine Temperatur zwischen 65 °C und 85 °C,
- Zugabe der gleichen Wassermenge,
- bei Erreichen der Phasenübergangstemperatur, Zugabe von Wasser, um die Emulsion zu erhalten.

## Revendications

1. Préparation à usage topique pour les traitements dermatologiques comprenant du cannabidiol synthétique comme principe actif principal,
le cannabidiol étant fourni en association avec un ou plusieurs ingrédients choisis dans le groupe des terpènes,
**caractérisée en ce que**
la préparation comprend en outre des huiles insaturées pouvant augmenter l'absorption cutanée du cannabidiol,
lesdites huiles comprenant un ou plusieurs composants huileux ozonisés.

2. Préparation selon la revendication 1, dans laquelle le cannabidiol est présent en un pourcentage compris entre 0,05 % et 0,5 % de son poids total.

3. Préparation selon l'une ou plusieurs des revendications précédentes, dans laquelle lesdits huiles comprennent de l'huile de neem ozonisée.

4. Préparation selon l'une ou plusieurs des revendications précédentes, dans laquelle le composant huileux ozonisé est présent en un pourcentage compris entre 0,01 % et 20 % de son poids total.

5. Préparation selon l'une ou plusieurs des revendications précédentes, dans laquelle ladite préparation se présente sous forme de gel, de crème, d'onguent, d'émulsion ou d'huile.

6. Procédé de préparation d'une préparation à usage topique pour des traitements dermatologiques **caractérisé en ce qu'**il comprend les étapes suivantes :
a) la solubilisation du cannabidiol synthétique à l'intérieur d'un composant huileux,
b) l'ajout d'un ou de plusieurs ingrédients choisis dans le groupe des terpènes,
c) l'ajout d'huiles insaturées comprenant un ou plusieurs composants huileux ozonisés.

7. Procédé selon la revendication 6, dans lequel les étapes b) et c) ont lieu à une température inférieure à 45°C.

8. Procédé selon la revendication 6 ou la revendication 7, dans lequel l'étape a) a lieu à une température comprise entre 30°C et 50°C.

9. Procédé selon une ou plusieurs des revendications précédentes 6 à 8, dans lequel, après l'étape a), les étapes suivantes sont prévues :
- la réalisation d'une émulsion,
- l'incorporation de la solubilisation obtenue à l'étape a) à l'intérieur de l'émulsion.

10. Procédé selon l'une ou plusieurs des revendications 6 à 9, dans lequel l'étape de fabrication d'une émulsion comprend les sous-étapes suivantes :
- le chauffage à une température comprise entre 65°C et 85°C d'une certaine quantité d'un composant lipophile,
- l'ajout de la même quantité d'eau,
- lorsque la température de transition de phase est atteinte, l'ajout d'eau afin d'obtenir l'émulsion.
